(19) Europäisches Patentamt
European Patent Office
Office européen des brevets

(11) EP 1 267 843 B1

(12) # EUROPÄISCHE PATENTSCHRIFT

(45) Veröffentlichungstag und Bekanntmachung des Hinweises auf die Patenterteilung:
**13.09.2006 Patentblatt 2006/37**

(21) Anmeldenummer: **01915410.3**

(22) Anmeldetag: **23.03.2001**

(51) Int Cl.:
***A61K 9/50*** *(2006.01)*

(86) Internationale Anmeldenummer:
**PCT/EP2001/003318**

(87) Internationale Veröffentlichungsnummer:
**WO 2001/074337 (11.10.2001 Gazette 2001/41)**

(54) **SYSTEM FÜR DEN TRANSPORT VON AKTIVSTOFFEN IN EINEM BIOLOGISCHEN SYSTEM**
SYSTEM FOR TRANSPORTING ACTIVE SUBSTANCES IN A BIOLOGICAL SYSTEM
SYSTEME DE TRANSPORT DE MATIERES ACTIVES DANS UN SYSTEME BIOLOGIQUE

(84) Benannte Vertragsstaaten:
**AT BE CH CY DE DK ES FI FR GB GR IE IT LI LU MC NL PT SE TR**

(30) Priorität: **03.04.2000 DE 10016559**

(43) Veröffentlichungstag der Anmeldung:
**02.01.2003 Patentblatt 2003/01**

(73) Patentinhaber: **EUCRO European Contract Research GmbH & Co. KG**
**41460 Neuss (DE)**

(72) Erfinder:
- **BLUM, Helmut**
**40595 Düsseldorf (DE)**
- **ROTH, Marcel**
**40589 Düsseldorf (DE)**
- **GREB, Wolfgang**
**40489 Düsseldorf (DE)**

(74) Vertreter: **Christophersen, Ruth et al**
**Christophersen & Partner**
**Patentanwälte**
**Feldstrasse 73**
**40479 Düsseldorf (DE)**

(56) Entgegenhaltungen:
**DE-A- 4 427 821** **DE-A- 19 612 001**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 267 843 B1

## Beschreibung

Die vorliegende Erfindung betrifft ein System für den Transport von Aktivstoffen in einem biologischen System, bestehend aus Aktivstoff sowie magnetische(n) Partikel(n), ein Verfahren zur Herstellung dieses Systems sowie die Verwendung des Systems zum Transport für pharmazeutische Wirk- und Aktivstoffe.

Unter Ferrofluiden versteht man magnetische Flüssigkeiten, in denen eine ferri- oder ferromagnetische Substanz, in der Regel Magnetit, als eine kolloidal dispergierte Phase in einem flüssigen Dispersionsmedium, wie Wasser, Paraffin, Toluol oder einer beliebigen anderen Flüssigkeit bis hin zu Quecksilber, dispergiert ist. Häufig wird ein Tensid oder Benetzungsmittel, wie Oleinsäure, einem solchen Gemisch zugefügt, um die Agglomerierung und somit die Bildung größerer Aggregate zu vermeiden. Aufgrund der geringen Größe der Kristallite verhalten sich die Teilchen superparamagnetisch, d.h. es ist keine Permanentmagnetisierung möglich.

Die Ferrofluide und auch andere metallische Kolloide sind seit vielen Jahren Gegenstand intensiver Forschung, da sie dazu in der Lage sind, Makromoleküle, insbesondere Proteine, zu binden.

Ein weiteres Einsatzgebiet der Ferrofluide ist, unerwünschte Zellen, insbesondere Krebszellen, aus Zellsuspensionen, zu entfernen. Beispielsweise bei der Behandlung von Krebs kann durch die Entfernung von Zellen eine Vielzahl von Problemen umgangen werden, wie der Einsatz von Toxinen, chemotherapeutischen Mitteln usw., wobei derartige Verfahren auf die physikalische Behandlung beschränkt sind. Die Dichte-Gradienten-Separation wurde zur Entfernung von Lymphocyten aus Knochenmarkzellen verwendet, jedoch ohne große Effizienz.

Eingesetzt werden unterschiedliche Arten von magnetischen Teilchen auch in Immunoassays, im Drug-Targeting und für Zellabtrennungen. Das am häufigsten verwendete magnetische Material ist Magnetit, das in eine Vielzahl von Trägersystemen oder Mikrosphären eingearbeitet wird oder direkt an Antikörper gebunden wird. Üblicherweise sind die magnetischen Partikel in entsprechende Hüllsubstanzen eingebettet, wie Polymere oder Kieselgele.

Aus der europäischen Patentanmeldung EP 0 156 537 sind magnetische kolloidale Flüssigkeiten bekannt, worin die magnetische Phase kolloidal in einem flüssigen Dispersionsmedium dispergiert ist. Die magnetische Phase umfasst feine magnetische Teilchen, die mit einem vernetzten, biologisch kompatiblem Polymer beschichtet sind.

In der deutschen Patentanmeldung DE 43 07 262 wird ein Verfahren zur Herstellung von magnetischem polymeren Siliciumdioxid beschrieben, worin magnetische Materialien, insbesondere Fe, $\beta$-$Fe_2O_3$, $\gamma$-$Fe_2O_3$, $Fe_3O_4$, in Alkalisilikatlösungen dispergiert oder als kolloidale Lösung zugegeben und mit Mineralsäuren oder Kohlendioxid gefällt und kondensiert werden. Auf die Oberfläche der erhaltenen Teilchen können Röntgenkontrastmittel aufgebracht werden. Sie werden bei bildgebenden Ultraschall- und NMR-Diagnostikverfahren eingesetzt sowie auch zur Anreicherung von radioaktiven Isotopen. Ein weiteres Einsatzgebiet ist der extra- oder intrakorporale Austrag oder die Bindung von biologischen Inhaltsstoffen, toxischen Substanzen, Bakterien oder Viren aus dem Organismus mit Hilfe von Magnetfeldern.

In der deutschen Patentanmeldung DE 43 25 386 wird eine magnetische Flüssigkeit (Ferrofluid) auf der Basis einer wässerigen Trägerflüssigkeit offenbart, worin magnetische Eisenoxidteilchen, die zum größten Teil aus Magnetit bestehen, durch eine erste monomolekulare Adsorptionsschicht aus gesättigten oder ungesättigten Fettsäuren und eine zweite Adsorptionsschicht aus oberflächenaktiven Substanzen stabilisiert sind. Sowohl die erste als auch die zweite Adsorptionsschicht bestehen aus Tensiden, die vollständig aus natürlich nachwachsenden Rohstoffen hergestellt sind. Die beschriebene wässerige magnetische Flüssigkeit kann in der Medizin als Markierungsstoff und/oder zum Transport von Wirkstoffen eingesetzt werden. DE 4427821 und DE 19612001 offenbaren ebenfalls superparamagnetische teilchen mit Oberflächen stabilisatorsubstanzen.

Eine Aufgabe der im Stand der Technik beschriebenen Partikel ist der zielgerichtete Transport von Stoffen, wie pharmazeutischen Wirkstoffen, im Körper und deren Anreicherung an gewünschten Stellen. Dieser Transport kann im wesentlichen mittels zweier Methoden erreicht werden, nämlich zum einen über die Anreicherung mittels an der Partikeloberfläche immobilisierter Antikörper oder mittels Permanentmagnetfeld.

Die bekannten eingesetzten magnetischen Partikel sind in der Regel Polymerpartikel mit Größen oberhalb 500 nm, die aus anorganischem oder organischem Polymer und eingelagerten magnetischen Teilchen bestehen. Kleinere Partikel werden üblicherweise nicht eingesetzt, da sich Partikel mit einer Teilchengröße unter 100 nm, sofern stabilisiert, kaum mittels eines Magnetfelds aufkonzentrieren lassen.

Ein wichtiges Einsatzgebiet der magnetischen bzw. superparamagnetischen Partikel im medizinischen Bereich ist die Chemotherapie zur Krebsbehandlung oder der Schutz von Implantaten mittels Antibiotika. Die Anbindung der Wirkstoffe erfolgt durch Quellung der Polymerpartikel oder durch Adsorption an die polymeren Oberflächen. Die magnetischen Teilchen können auch in wässerigem Medium suspendiert eingesetzt werden, wobei an deren Oberfläche üblicherweise oberflächenaktive Substanzen angelagert sind, welche die magnetischen Teilchen sowie gegebenenfalls daran adsorbierte Wirkstoffe in Suspension halten.

Wie bereits beschrieben, weisen die aus dem Stand der Technik bekannten magnetischen Partikel, die zur Anreicherung oder zum zielgerichteten Transport von Stoffen in biologischen Systemen eingesetzt werden, eine modifizierte Oberfläche auf. Die Modifikation der Oberfläche dient dazu, diese Stoffe in Suspension zu halten und die Aktivstoffe zu binden. Die Modifizierung der Oberflächen hat den Nachteil, dass diese Modifizierung ein zusätzlicher Arbeitsschritt bei der Herstellung darstellt. Ferner besteht die Gefahr, dass die zur Modifikation der Oberflächen eingesetzten Polymere mit den Aktivstoffen und gegebenenfalls auch mit im biologischen System, d.h. im Körper, befindlichen Proteinen etc. Wechselwirkungen eingehen können, die die Wirksamkeit der Aktivstoffe beeinträchtigt und in manchen Fällen sogar zu unerwünschten, ggf. toxischen Nebenreaktionen und Wirkungen dieser Mittel führen kann.

Ein weiterer Nachteil ist, dass das nachträgliche Aufbringen einer Beschichtung auf bereits oberflächenmodifizierte Partikel aufwendig und daher zeitintensiv ist. Schließlich weisen die beschichteten Partikel eine relativ geringe Adsorptionskapazität für pharmazeutische Wirkstoffe auf.

In der EP 0 275 285 B1 wird ein Verfahren zur Herstellung eines stabilen superparamagnetischen Fluids beschrieben, in dem das Dispergieren und Stabilisieren durch Einsatz von Ultraschall erfolgt. Die Ultraschallbehandlung hat den Nachteil, dass durch diesen Energieeintrag ggf. auf den Partikeln aufgebrachte, thermisch oder mechanisch instabile Substanzen zerstört werden.

Der vorliegenden Erfindung lag die Aufgabe zugrunde, ein System für den Transport von Aktivstoffen in einem biologischen System zur Verfügung zu stellen, worin eine Modifizierung der magnetischen Partikel nicht unabdingbar erforderlich ist und das eine Einarbeitung dieser Partikel sowohl in wässerige und ölige Suspensionen als auch in Mikroemulsionen, Öl-in-Wasser-Emulsionen, Wasser-in-Öl-Emulsionen und auch Wasser-in-Öl-in-Wasser-Emulsionen ermöglicht.

Gegenstand der vorliegenden Erfindung ist demgemäß ein Stabilisator-freies System für den Transport von Aktivstoffen in einem biologischen System bestehend aus einem oder mehreren Aktivstoff(en) sowie magnetischen Partikeln, das dadurch gekennzeichnet ist, dass die Partikel auf zumindest einem Teil ihrer Oberfläche mit Aktivstoff (en) versehen sind.

Stabilisator-frei bedeutet in der vorliegenden Erfindung, dass die magnetischen Partikel ohne Zusatz von Hilfsmitteln, wie Emulgatoren oder Oberflächenbeschichtungen, wie sie im Stand der Technik beschrieben werden, mit Aktivstoffen beaufschlagt sind bzw. von diesen umhüllt sind. Auch ist eine Behandlung von mit Aktivstoffen beladenen Partikeln nicht erforderlich und vorzugsweise ausgeschlossen. In der einfachsten Ausgestaltung der vorliegenden Erfindung besteht das erfindungsgemäße System aus magnetischem Partikel und Aktivstoff.

Erfindungsgemäß ist zumindest ein Teil der Oberfläche der magnetischen Partikel mit Aktivstoff(en) versehen. Das bedeutet, dass die Aktivstoffmoleküle unmittelbar auf der Oberfläche der Partikel aufgebracht sind. Die Partikel können auch vom Aktivstoff umhüllt sein, was zum Beispiel dann der Fall ist, wenn die Aktivstoffe aufgrund ihrer Struktur, Form oder Größe die magnetischen Partikel umgeben aber nicht auf der Oberfläche direkt aufgebracht sind. Eine Umhüllung liegt beispielsweise dann vor, wenn als Aktivstoffe Zellen, Zellkulturen bzw. Zellbestandteile verwendet werden und die magnetische Partikel im inneren der Zellen, Zellkulturen bzw. Zellbestandteile vorliegen, oder wenn die Aktivstoffe aufgrund ihrer Molekülgröße die Struktur eines Knäuels aufweisen, in dessen Inneren sich die magnetischen Partikel befinden.

Das erfindungsgemäße System kann zum zielgerichteten Transport von Aktivstoffen an einen speziellen Wirkort im Körper bzw. biologischen System als auch zum "Abtransport" von unerwünschten Komponenten im/am Körper oder aus dem Körper eingesetzt werden. Es hat den Vorteil, dass die Aktivstoffe unmittelbar auf den Partikeln aufgebracht sind, das erfindungsgemäße System im einfachsten Fall also aus Aktivstoff(en) und magnetischen Partikeln besteht.

Es wurde festgestellt, dass durch die gezielte Beladung mit Aktivstoff zum einen eine Art Carrierfunktion der Partikel im umgebenden Medium erzielt wird und zum anderen kann durch Anlegen eines Magnetfelds eine selektive Anreicherung der beladenen Partikel (Sedimentation) und Anreicherung der Partikel erreicht werden. Eine im Vergleich zu größeren Partikeln relativ große Partikeloberfläche ermöglicht eine höhere Beladung der Oberfläche mit Aktivstoffen, d.h. das erfindungsgemäße System kann im Vergleich zum Stand der Technik eine deutlich höhere Konzentration an Aktivstoffen aufnehmen. Insgesamt können mit weniger magnetischem Material gleiche Aktivstoffgehalte in das biologische System eingebracht werden.

Biologisches System in Sinne der vorliegenden Erfindung bedeutet sowohl der menschliche oder tierische Körper selbst aber auch ein extrakorporales System, wie z.B. aus dem Körper eluierte Zellen/Zellkulturen und/oder außerhalb des Körpers befindliche Geräte, in denen Körperflüssigkeiten gereinigt, werden. Die erfindungsgemäßen Partikel werden üblicherweise von den Organen, Geweben und Zellen sowie Implantaten aufgenommen.

Als magnetische Partikel werden superparamagnetische Partikel eingesetzt, ausgewählt aus Metalloxide und/ oder Metalle. Superparamagnetische Teilchen besitzen keine Remanenz, d.h. sie lassen sich in einem magnetischen Gradientenfeld reversibel bewegen und konzentrieren.

Der Vorteil der eingesetzten magnetischen Partikel besteht insbesondere darin, dass sie vollständig aus anorganischem Material aufgebaut sind und sich gut im Magnetfeld sedimentieren lassen. Für den Einsatz der Partikel zum Transport von Aktivstoffen im biologischen System sind keine weiteren Komponenten für die Modifikation der Partikel selbst erforderlich, wie Beschichtung mit Polymeren etc. Das System kann für den jeweiligen Anwendungszweck beliebig konfektioniert bzw. eingestellt werden.

Beispiele für geeignete magnetische Partikel sind $\gamma$-$Fe_2O_3$, $Fe_3O_4$, $MnFe_2O_4$, $NiFe_2O_4$, $CoFe_2O_4$ und deren beliebigen Gemische, wobei $Fe_3O_4$ (Magnetit) besonders bevorzugt eingesetzt wird. Als mögliche Metalle sind Fe, Co,

Ni sowie deren Legierung ggf. auch mit anderen Metallen zu nennen.

Die erfindungsgemäß eingesetzten magnetischen Partikel weisen vorzugsweise eine Teilchengröße von 1 bis 300 nm, vorzugsweise bis 100 nm auf, wobei hier die einzelnen diskreten Kristallite gemeint sind. Es können auch Agglomerate vorliegen, deren Gesamtteilchengröße oberhalb von 100 nm, insbesondere oberhalb von 300 nm liegt.

Die volumengewichtete mittlere Kristallitgröße ist mit Röntgenbeugungsverfahren, insbesondere über eine Scherrer-Analyse, bestimmbar. Das Verfahren ist beispielsweise beschrieben in: C. E. Krill, R. Birringer: "Measuring average grain sizes in nanocrystalline materials", Phil. Mag. A 77, S. 621. (1998). Demnach kann die volumengewichtete mittlere Kristallitgröße D bestimmt werden durch den Zusammenhang

$$D = K\lambda/\beta\cos\theta.$$

Dabei ist $\lambda$, die Wellenlänge der verwendeten Röntgenstrahlung, $\beta$ ist die volle Breite auf halber Höhe des Reflexes an der Beugungsposition $2\theta$. K ist eine Konstante der Größenordnung 1, deren genauer Wert von der Kristallform abhängt. Man kann diese Unbestimmtheit von K vermeiden, indem man die Linienverbreiterung als integrale Weite $\beta_i$ bestimmt, wobei $\beta$, definiert ist als die Fläche unter dem Röntgenbeugungsreflex geteilt durch dessen maximaler Intensität $I_0$:

$$\beta_i = 1/I_0 \int_{2\theta_1}^{2\theta_2} I(2\theta)d(2\theta)$$

Dabei sind die Größen $2\theta_1$ und $2\theta_2$ die minimale und maximale Winkelposition des Bragg-Reflexes auf der $2\theta$-Achse. $I(2\theta)$ ist die gemessene Intensität des Reflexes als Funktion von $2\ \theta$. Unter Verwendung von diesem Zusammenhang ergibt sich als Gleichung zur Bestimmung der volumengewichteten mittleren Kristallitgröße D: $D = \lambda/\beta_i\cos\theta$.

In einer möglichen Ausführungsform der vorliegenden Erfindung werden die magnetischen Partikel als Nanopartikel mit einer Teilchengröße von vorzugsweise unter 100 nm eingesetzt. Auf diese Teilchen kann der eingesetzte Aktivstoff adsorbiert werden, wobei es sich als besonders vorteilhaft erwiesen hat, wenn der Aktivstoff bereits bei der Bildung der magnetischen Partikel, z.B. durch größenkontrollierte Fällung im wässerigen Medium mittels alkalischer Substanzen oder durch Reduktion von Metallkationen vorliegt. Durch die in situ erzeugte große Partikeloberfläche kann eine optimale Adsorption des Aktivstoffes an die Oberfläche mittels funktioneller, vorzugsweise ionischer oder polarer Gruppen im Aktivstoffmolekül, wie OH-, SH-, Hydroxid-, Amino-, Carboxyl-, Ether-, Sulfo-, Phosphonsäuregruppen usw., erfolgen. Es ist auch möglich, den Aktivstoff nachträglich auf die gefällten Partikel aufzubringen, z.B. durch Suspension der ungecoateten (nicht modifizierten) magnetischen Partikel in einer den Aktivstoff beziehungsweise das Aktivstoffgemisch enthaltenden flüssigen Phase, vorzugsweise Wasser.

In einer weiteren möglich Ausführungsform der vorliegenden Erfindung können die Aktivstoffe auch über sog. Spacer-Gruppen an die magnetischen Partikel gebunden werden. Spacer sind kurze organische Molekül-Ketten, die bei der Immobilisierung von Molekülen auf Trägern genutzt werden, wobei die Spacer-Moleküle keine Beschichtung darstellen. Spacer können beispielsweise eingesetzt werden, wenn die Aktivstoffe keine polaren Gruppen oder ionischen Gruppen aufweisen. Die Spacer-Moleküle können die Bindung zwischen magnetischen Partikeln und den Aktivstoffen verbessern. Sie weisen vorzugsweise eine oder mehrere polare Gruppe(n) auf. Als Beispiele kann auf die bereits genannten Gruppen verwiesen werden. Insbesondere wenn kationische Aktivstoffe verwendet werden, haben sich Spacer mit zwei polaren Gruppen, wie Aminocarbonsäuren, Diamine, Betaine, Dicarbonsäuren, Aminophosphonate, etc. als geeignet erwiesen.

In einer weiteren Ausführungsform werden sogenannte Agglomerate von magnetischen Partikeln eingesetzt, die aus Agglomeraten von Nanopartikeln, d.h. von Kristalliten mit einer Teilchengröße unter 100 nm, bestehen. Diese Agglomerate können aus einzelnen Kristalliten bestehen, welche an ihrer Kontaktfläche entweder reversibel agglomeriert oder irreversibel durch Koaleszens, d.h. durch Zusammenwachsen über die Korngrenzen hinweg, agglomeriert sind. Ein Vorteil von Agglomeraten besteht darin, dass sie sowohl eine äußere als auch eine innere Oberfläche, d.h. Hohlräume aufweisen, so dass die Aktivstoffe innen und außen gebunden werden können. Agglomerate können beispielsweise erhalten werden, indem die magnetischen Partikel in Abwesenheit eines Wirkstoffs gefällt werden, durch Trocknung oder Gefriertrocknung wirkstofffreier oder mit Wirkstoff beladenen Nanopartikel mit anschließender Redispergierung,

Agglomeratbildung, welche durch die Synthesebedingungen gesteuert werden kann, wie Temperaturerhöhung, Einstellung des pH-Wertes, hoher Elektrolytgehalt oder durch eine geeignete Nachbehandlung der gefällten Partikel bei Temperaturen von über 100°C.

Aktivstoffe im Sinne der vorliegenden Erfindung sind sowohl Stoffe, die in den Körper eingebracht werden, als auch Stoffe, die aus ihm entfernt werden sollen, z.B. synthetische pharmazeutische Wirkstoffe, natürliche pharmazeutische Wirkstoffe und Extrakte, natürliche und rekombinante Peptide, Proteine, Enzyme, Antikörper und Antikörperfragmente, endogene biologische Einheiten wie lebende und abgestorbene Zellen, Zellbestandteile und Organellen, synthetische und natürliche DNA, Gene, Chromosomen, gentechnisch veränderte autologe oder heterologe Zellen, und xenobiotische Einheiten wie Bakterien, Viren, Mycoplasma, Pilze, und Sporen, wärmeleitfähige Substanzen, wie Metalle, radiologisch wirksame Stoffe, wie $\gamma$-Strahler, Aktivstoffe enthaltende partikuläre exogene Einheiten wie Liposomen, Mikrokapseln und Nanopartikel, sowie beliebige Gemische der voranstehenden.

In einer besonders bevorzugten Ausführungsform dieser Ausgestaltung sind die Aktivstoffe ausgewählt aus wasserlöslichen und/oder lipidlöslichen pharmazeutischen Wirkstoffen.

In einer bevorzugten Ausführungsform der vorliegenden Erfindung werden als Aktivstoffe geminale Bisphosphonsäuren und/oder deren physiologisch verträglichen Salze, vorzugsweise solche mit der allgemeinen Formel I eingesetzt:

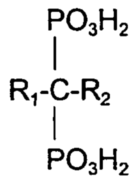

Formel I

in der ist

$R_1$ ein linearer oder verzweigter Alkylrest mit 1 bis 10 Kohlenstoffatomen, der gegebenenfalls substituiert sein kann durch Substituenten wie Aminogruppen, N-Mono- bzw. N-Dialkylaminogruppen, wobei die Alkylgruppen 1 bis 5 C-Atome und/oder SH-Gruppen enthalten können, oder ein substituierter oder unsubstituierter carbo- oder heterocyclischer Arylrest, der ggf. ein oder mehrere Heteroatome und als Substituenten verzweigte und unverzweigte Alkylreste mit 1 bis 6 C-Atomen, freie oder mono- resp. dialkylierte Aminogruppen mit 1 bis 6 C-Atomen oder Halogenatome aufweisen kann, und

$R_2$ gleich OH, ein Halogenatom, vorzugsweise Cl, H oder $NH_2$.

Als Beispiele für geeignete Salze der Verbindungen mit der Formel I können Alkalimetallsalze, Ammoniumsalze und Ethanolamminsalze genannt werden.

Derartige Verbindungen eignen sich insbesondere für die Behandlung von osteoporotischen Erkrankungen, wobei folgende Verbindungen besonders bevorzugt sind:

3-(Methyl-pentylamino)-1-hydroxypropan-1,1-diphosphonsäure (Ibandronsäure),
1-Hydroxyethan-1,1-diphosphonsäure (Etidronsäure),
Dichlormethandiphosphonsäure (Clodronsäure),
3-Amino-1-hydroxypropan-1,1-diphosphonsäure (Pamidronsäure),
4-Amino-1-hydroxybutan-1,1-diphosphonsäure (Alendronsäure),
2-(3-Pyridin)-1-hydroxyethan-1,1-diphosphonsäure (Risedronsäure),
4-Chlorphenylthiomethan-1,1-di-phosphonsäure (Tiludronsäure),
Pyrimidinyl-1-hydroxyethan-1,1-diphosphonsäure (Zoledronsäure),
Cycloheptylaminomethan-1,1-diphosphonsäure (Cimadronsäure),
6-Amino-1-hydroxyhexan-1,1-diphosphonsäure (Neridronsäure),
3-(N,N-Dimethylamino)-1-hydroxypropan-1,1-diphosphonsäure (Olpadronsäure),
3-Pyrrol-1-hydroxypropan-1,1-diphosphonsäure und/oder
2-Pyrimidazol-1-hydroxyethan-1,1-diphosphonsäure (Minodronsäure)

sowie deren physiologisch verträglichen Salzen.

Wie bereits voranstehend ausgeführt besteht das erfindungsgemäße System in seiner einfachsten Form aus magnetischem Partikel(n) und einem oder mehreren Aktivstoff(en). Dieses System kann in an sich bekannter Weise in

eine pharmazeutische Zubereitung für die orale, parenterale, intravenöse, inhalative und/oder topische Applikation überführt werden und dem biologischen System zuführt werden. Als geeignete Formen der pharmazeutischen Zubereitung sind Suspensionen, Emulsionen und liposomale Systeme zu nennen.

In einer möglichen Ausführungsform liegt das erfindungsgemäße System in Form einer Suspension vor. Als Suspensionsmedium ist Wasser oder physiologische NaCl-Lösung bevorzugt.

In einer weiteren Ausgestaltung der vorliegenden Erfindung liegt das erfindungsgemäße System für den Transport von Aktivstoffen als Emulsion vor, wobei sowohl Wasser-in-Öl- als auch Öl-in-Wasser-Emulsionen möglich sind. Die magnetischen Partikel liegen vorzugsweise ausschließlich in der Öl-Phase vor, welche die innere Phase, d.h. die Tröpfchen bildet. Zusätzlich können auch magnetische Partikel in der Wasser-Phase angereichert sein. In Fällen, in denen die magnetischen Partikel in der Öl-Phase vorliegen, spricht man auch von Ölferrofluiden. Es können sowohl Makroemulsionen als auch Mikroemulsionen eingesetzt werden, d.h. thermodynamisch stabile Emulsionssysteme mit Tröpfchengrößen < 500 nm.

Ölferrofluide können als Träger für lipidlösliche Aktivstoffe dienen, so dass sowohl die magnetisierbaren Partikel als auch der bzw. die Aktivstoffe in der Öl-Phase vorliegen. In einer weiteren Ausgestaltung werden wasserlösliche Aktivstoffe eingesetzt, die in gelöster Form in der wässerigen Phase vorliegen, und die magnetischen Partikel in der Öl-Phase vorliegen.

Um eine Erhöhung der physiologischen Verträglichkeit und eine schnelle Verteilung der Aktivstoffe im Blutstrom zu bewirken, kann eine Emulgierung der beladenen Partikel in Wasser gegebenenfalls auch unter Verwendung geeigneter physiologisch verträglicher Emulgatoren erfolgen. Ein Beispiel für einen handelsüblichen Emulgator ist Solutol® (BASF AG). Liegen der Aktivstoff und die magnetischen Partikel in der Öl-Phase vor, so können auch hier die Wirkstoffe an die magnetischen Partikel gebunden sein (adsorbiert sein), was jedoch nicht unbedingt erforderlich ist.

In einer weiteren Ausgestaltung können wasserlösliche Wirkstoffe in der Wasser-Phase einer Ölferrofluid-Emulsion gelöst oder suspendiert werden. Diese Ausführungsform ist besonders geeignet, wenn die Aktivstoffe wasserlösliche Polymere, wie Zellbestandteile, Proteine etc. sind. Unter Zuhilfenahme geeigneter Emulgatoren kann eine derartige Aktivstoffe enthaltende Wasserphase in eine magnetisches Öl enthaltende Emulsion überführt werden. Das Emulsionsgemisch kann anschließend mittels Permanentmagnetfeld am Wirkort aufkonzentriert werden.

In einer bevorzugten Ausführung werden die unabhängig voneinander in der wässerigen Phase vorliegenden Aktivstoffe und magnetischen Partikel in den wässerigen Innenraum von Liposomen eingeschlossen. Falls erwünscht kann die nicht-eingeschlossene Fraktion durch Zentrifugation oder Gelchromatographie abgetrennt werden, woraus ein gereinigtes magnetisches Liposomenprodukt resultiert. Derselbe Prozess kann angewandt werden, um oben beschriebene magnetischen Partikel mit adsorbierten Aktivstoffen liposomal zu verkapseln.

In einer möglichen Ausführungsform der vorliegenden Erfindung sind die Aktivstoffe auf den magnetischen Partikeln adsorbiert und liegen gleichzeitig in freier Form in der wässerigen Phase und/oder in der Öl-Phase vor. Die Adsorption erfolgt entweder während der Bildung der magnetischen Partikel durch Fällung oder durch Suspendieren der magnetischen Partikel in einer Lösung, einer Suspension bzw. einer Dispersion der Aktivstoffe. Die beladenen Partikel können jeweils in Form einer Suspension, Mikroemulsion, Öl-in-Wasser-Emulsion, Wasser-in-Öl-Emulsion, Wasser-in-Öl-in-Wasser-Emulsion etc. formuliert werden. Die beladenen magnetischen Partikel können in der wässerigen Phase oder in der Öl-Phase angereichert sein.

Die Herstellung des erfindungsgemäßen Systems für den Transport von Aktivstoffen in einem biologischen System kann auf verschiedene Arten erfolgen.

In einer ersten Ausführungsform werden ein wasserlöslicher oder in Wasser suspendierbarer Aktivstoff und eine in Wasser lösliche Vorstufe der magnetischen Partikel in Wasser gelöst und die magnetischen Partikel durch Ausfällung gebildet, wobei die magnetischen Partikel beladen mit dem Aktivstoff als Feststoff ausfallen.

In einer weiteren Ausgestaltung dieses Herstellungsverfahrens werden die magnetischen Partikel in eine Lösung oder Suspension des Aktivstoffs in Wasser oder einer anderen Flüssigkeit gegeben. Das Beladen der magnetischen Partikel mit Aktivstoff erfolgt durch Adsorption auf der Oberfläche der magnetischen Partikel.

In einer weiteren Ausgestaltung können ölbasierte Ferrofluide erhalten werden. Vorzugsweise werden die magnetischen Partikel zunächst mit einem festen oder flüssigen Öl oder geschmolzenem Wachs unter Rühren und ggf. unter Erwärmen vermischt. Anschließend können die erhaltenen lipidlöslichen Partikel in Gegenwart des Aktivstoffes in an sich bekannter Weise in Wasser emulgiert werden.

Als Öle oder Wachse können alle auf dem jeweiligen Einsatzgebiet geeigneten und bei Verarbeitungstemperatur flüssigen natürlichen oder sythetischen Öl oder Wachse eingesetzt werden, sofern sie pharmazeutisch unbedenklich sind. Sofern die Öle oder Wachse in fester Form vorliegen, können sie zur Herstellung eines ölbasierten Ferrofluids erwärmt werden.

In einer weiteren Ausgestaltung des Herstellungsverfahrens können lipidlösliche Aktivstoffe in dem erfindungsgemäßen System eingesetzt werden. In einer bevorzugten Ausgestaltung werden diese Aktivstoffe zunächst mit den erhaltenen lipidlöslichen Partikeln vermischt und das Gemisch wird anschließen in Wasser emulgiert. In einer weiteren möglichen Ausgestaltung können die lipidlöslichen Aktivstoffe zu den magnetischen Partikeln zugesetzt werden bevor

oder während diese mit dem festen oder flüssigen Öl oder Wachs vermischt werden.

In einer bevorzugten Ausgestaltung werden diese Aktivstoffe zunächst mit den erhaltenen lipidlöslichen Partikeln vermischt und das Gemisch wird anschließen in Wasser emulgiert.

Ein weiterer Gegenstand der vorliegenden Erfindung betrifft die Verwendung des oben beschriebenen Systems zur Herstellung eines Arzneimittels für den Transport von Aktivstoffen in einem biologischen System für den zielgerichtete Transport pharmazeutischen Wirkstoffen in dem biologischen System sowie die Verwendung des Systems zur Herstellung eines Arzneimittels für die Anreicherung von Aktivstoffen in dem biologischen System an vorgegebenen Orten. Zu diesem Zweck kann das Ferrofluid-Arzneistoffprodukt in geeignete konventionelle Arzneiformen überführt oder eingearbeitet werden. Z.B. eignet sich für die orale Verabreichung eines mit Arzneistoff beladenen Ölferrofluids die Arzneiform der Soft-Gelatine Kapsel. Für die systemische Injektion oder die inhalative Anwendung eignet sich besonders die Form der liposomalen Verkapslung wie oben beschrieben. Zur Einbringung und Positionierung in Körperhöhlen wie Peritoneum, Blasenraum, Urogenital- und Vaginaltrakt sind wässerige Suspensionen oder Öl-in-Wasser-Emulsionen besonders geeignet. Für den gezielten Transport der verwendeten Aktivstoffe kann beispielsweise extern ein Magnetfeld an oder in der Nähe des zu behandelnden "Ortes" angelegt werden, wodurch der Aktivstoff an der gewünschten Stelle lokal konzentriert werden kann (Drug-targeting).

Die Aktivstoffe können am Zielorgan bzw. Zielort ihre Wirkung entsprechend ihrer Aktivität entfalten und ggf. von den Partikel freigesetzt werden.

Aktivstoffe, die ihre Wirkung unmittelbar durch Kontakt mit dem zu behandelnden biologischen System entfalten sollen, werden vorzugsweise unmittelbar am Wirkort freigesetzt. Ein Beispiel für derartige Aktivstoffe ist die Wirkung von Chemotherapeutika, Cytostatika, die Therapie unterstützenden Wirkstoffen, wie entzündungshemmenden Mitteln, Schmerzmittel, etc., die mittels angelegtem Magnetfeld durch Transport über die magnetischen Partikel zu ihrem Wirkort transportiert werden, dort aufgrund ihrer Affinität zum behandelnden Gewebe, Tumor o. ä. freigesetzt werden und ihre Wirkung entfalten. Nach Beendigung der Freisetzung können die magnetischen Partikel wieder über das angelegte Magnetfeld, das bedeutet nach Verändern der Position des Magnetfelds, aus dem Körper entfernt werden.

Werden beispielsweise die voranstehend beschriebenen Bisphosphonate als Aktivstoffe verwendet, so kann bei der Behandlung von Knochentumoren bzw. Metastasen im Knochen, das erfindungsgemäße System, ein Ferrofluid aus magnetischem Partikeln und Bisphonat, zum Tumor transportiert werden. Das Bisphosphonat wird an das Knochenapatit gebunden und inhibiert den Knochenumbau. Durch das externe Magnetfeld werden die Tumorzellen lokal erhitzt und so zerstört.

In einer weiteren Ausführungsform können Aktivstoffe, die radiologische Eigenschaften zeigen, wie $\gamma$-Strahler etc., erfindungsgemäß zu ihrem Wirkort transportiert werden, dort das zu behandelnde Gewebe, gegebenenfalls mit externer Erwärmung, durch lokale Bestrahlung zerstören. Zum Abschluss der Bestrahlung können die Partikel mittels eines Magnetfelds wieder entfernt werden.

Neben dem Targeting-Effekt hat der Einsatz der magnetischen Partikel hat den weiteren Vorteil, daß die Partikel durch die andauernde Magnetisierung die Freisetzung der Aktivstoffe gefördert wird. Durch die andauernde Magnetisierung kann eine lokale Überwärmung entstehen, wodurch die Freisetzung von Aktivstoffen, die nur lose auf der Oberfläche der Partikel aufgebracht sind, unterstützt wird. Ein weiterer Freisetzungsmechanismus, der insbesondere dann auftritt, wenn keine Dauermagnetisierung vorliegt, ist die langsame Dissoziation des Aktivstoffes vom magnetischen Partikel. Diese Freisetzung kann über eine chemische, insbesondere enzymatische, hydrolytische oder thermische Abspaltung oder auch über eine rein physikalische Abspaltung erfolgen. Die thermische Abspaltung der Aktivstoffe von den magnetischen Partikeln wird vorzugsweise durch ein angelegtes Magnetfeld unterstüzt.

Ein weiterer Vorteil der bei der andauernden Magnetisierung der am lokalen Wirkort befindlichen magnetischen Partikel ist die auftretende lokale Überwärmung. Diese kann genutzt werden, um erkrankte Gewebe beziehungsweise Tumorgewebe zu zerstören. Die lokale Überwärmung mittels Einsatz von magnetischem Feld wird auch als lokale oder zelluläre Hyperthermie bezeichnet.

Die Kombination aus dem erfindungsgemäßen System und der Hyperthermie läßt sich beispielsweise auch bei der Behandlung von Tumoren, die unter Verwendung von sogenannten Thermo-seeds behandelt werden können, einsetzen. Die Thermo-seeds bestehen in der Regel aus einer Legierung aus einem magnetischem Metall, wie Eisen oder Kobalt, und einem nichtmagnetischen Material, wie den Edelmetallen Gold, Silber, Palladium oder Platin. Nach Implantation der Thermo-seeds in den Tumor kann das erfindungsgemäße System, das beispielsweise magnetische Partikel und ein Chemotherapeutikum als Aktivstoff enthalten, verabreichen. Das erfindungsgemäße System reichert sich in Thermo-seeds an und bildet so ein lokales Cytostatikumdepot. Im Anschluss an die Anreicherung des erfindungsgemäßen Systems am Tumor kann die übliche Thermo-seed-Behandlung erfolgen, indem außerhalb des Körpers ein magnetisches Wechselfeld abgestrahlt wird, welches zu einer Erhitzung der Thermo-seeds und die damit verbundene Zerstörung der Tumorzellen führt.

Eine ähnliche tumorbehandelnde Wirkung kann erreicht werden, wenn als Aktivstoff ein Metall mit einer guten Wärmeleitfähigkeit, wie Palladium oder Platin verwendet wird. In dieser Ausführungsform wird das erfindungsgemäße System aus magnetischen Partikel und Metall an ihren Wirkort transportiert, anschließend erfolgt eine Erwärmung auf

den Curie-Punkt durch externes Anlegen eines magnetischen Wechselfeldes. Die auftretende limitierte Überwärmung führt zur Zerstörung der Tumorzellen. Diese Ausführungsform kann auf jeden beliebigen Tumor angewendet werden. Das erfindungsgemäße System kann in einer geeigneten pharmazeutischen Zubereitung wie oben beschrieben injiziert oder auch inhaliert werden. Beispielsweise ist die Inhalation dieses Systems zur Behandlung von Lungentumoren geeignet.

## Beispiele

Beispiel 1: Herstellung eines wasserdispergierten Ferrofluids

6.48g $FeCl_3$ wurden in 40 g entionisiertem Wasser gelöst. Außerdem wurden 3,97 g $FeCl_2$*$4H_2O$ in einer Mischung aus 8 ml entionisiertem Wasser und 2 ml 37%iger Salzsäure gelöst. Kurz vor Einsatz der Lösungen im Fällungsprozess wurden die beiden Mischungen vereinigt.

In einem Becherglas wurden 400 ml entionisiertes Wasser mit 10 g NaOH und 0,2 g Hydroxyethandiphosphonsäure (HEDP) verrührt. Nach Abkühlen wurden hierzu unter starkem Rühren die salzsaure Eisensalzlösung gegossen. Mittels Magnetfeld wurde der gebildete schwarze Niederschlag sedimentiert und die überstehende Lösung abdekantiert. Anschließend wurde das gefällte Material mehrmals in Wasser aufgenommen und dekantiert, um Fremdionen zu entfernen. Anschließend wurden 0,5 g HEDP und 100 ml Wasser zugegeben. Nach 1 stündigem Rühren bei 40 °C wurde 12 h lang bei RT nachgerührt. Nicht suspendierte Anteile wurden durch Zentrifugieren (5000-11000 Umdrehungen/ Minute) abgetrennt. Auf diese Weise wurde eine magnetische Flüssigkeit erhalten, die im Rotationsverdampfer bis zum Erhalt des gewünschten Feststoffgehalts eingeengt wurde.

Beispiel 2: Herstellung einer Ölferrofluid-in-Wasser-Emulsion

a. Herstellung des Ölferrofluids

7.8 g wasserfreies Eisen(III)-chlorid wurden in 50g $CO_2$-freiem Wasser gelöst. Gleichzeitig wurden in einem zweiten Gefäß 4.8g $FeCl_2$•$4H_2O$ in 10g Wasser gelöst und mit Salzsäure bis zu einem pH-Wert von 2 angesäuert. Beide Lösungen wurden dann vereinigt und zu einer kräftig gerührten Vorlage, bestehend aus 100 ml 25%iger Ammoniaklösung und 300 ml entionisiertem Wasser unter Ausfällung eines schwarzen Niederschlags gegeben. Nach mehrmaligem Waschen mit Wasser und jeweiligem Abzentrifugieren und Abdekantieren überstehender wässeriger Phase wurde der Niederschlag mit 100g Wasser und 2.0g Laurinsäure versetzt. Unter Rühren wurde auf 85 °C erwärmt, bis der Niederschlag unter Flockenbildung sedimentierte. Anschließend wurde zu der noch 85°C heißen Mischung 10g Sonnenblumenöl gegeben und eine Stunde gerührt. Hierbei ging der Niederschlag dispersionsstabil in die Ölphase über, welche abgetrennt und mehrmals mit Wasser ausgeschüttelt wurde. Es wurde ein ölbasiertes Ferrofluid erhalten.

b. Transport eines Aktivstoffs zu einem befallenen Gelenk (Aktivstoff: Antirheumatikum Nabumeton)

Das erhaltene Ölferrofluid wurde mit isotonischer Salzlösung im Volumenverhältnis 1: 9 vermischt. Durch Zugabe des Emulgators Solutol® HS 15 (Polyethylenglykol 660-12-Hydroxystearat, Hersteller: BASF AG) mit einem Gewichtsanteil von 15g pro Liter und anschießendem Rühren mittels Magnetrührer wurde eine Ferrofluid-in-Wasser-Emulsion erhalten mit Tröpfchengrößen im Bereich 10 - 100 μm. Die Beladung mit dem öllöslichen Wirkstoff (Nabumeton) erfolgte vor der Emulgierung durch Lösen von 2 ml Wirkstoff in 8 ml ölbasiertem Ferrofluid.

Beispiel 3: Herstellung eines Ferrofluids, worin der Aktivstoff der Ölphase entspricht

7.8 g wasserfreies Eisen(III)-chlorid wurden in 50 g $CO_2$-freiem Wasser gelöst. In einem zweiten Gefäß wurden 4.8 g $FeCl_2$*$4H_2O$ in 10 g Wasser gelöst, die erhaltene Lösung wurde mit Salzsäure bis zu einem pH-Wert von 2 angesäuert. Beide Lösungen wurden zu einer Mischung vereinigt und zu einer stark gerührten Vorlage, bestehend aus 100 ml 25% Ammoniaklösung und 300 ml entionisiertem Wasser unter Ausfällung eines schwarzen Niederschlags gegeben. Nach mehrmaligem Waschen mit Wasser und jeweiliger Abzentrifugation und Abdekantieren überstehender wässeriger Phase wurde der Niederschlag mit 100 g Wasser und 2.0 g Laurinsäure versetzt. Unter Rühren erwärmte man auf 85 °C, bis der Niederschlag unter Flockenbildung sedimentierte. Anschließend wurden zu der noch 85°C heißen Mischung 10 g des öllöslichen Wirkstoffes Nabumeton gegeben und eine Stunde gerührt. Hierbei ging der Niederschlag dispersionsstabil in die Ölphase über, welche abgetrennt und mehrmals mit Wasser ausgeschüttelt wurde. Man erhielt ein ölbasiertes Ferrofluid.

Das Ölferrofluid wurde mit isotonischer Salzlösung im Volumenverhältnis 1 : 9 vermischt. Durch Zugabe des Emulgators Solutol HS 15 (Polyethylenglykol 660-12-Hydroxystearat, Hersteller: BASF AG) mit einem Gewichtsanteil

von 15g pro Liter und anschießendes Rühren mittels Magnetrührer wurde eine Ferrofluid-in-Wasser-Emulsion erhalten mit Tröpfchengrößen im Bereich 5-100 Mikrometer. Zur Wirkstoffdosierung wurde die Ölphase entsprechend mit isotonischer Salzlösung verdünnt.

Beispiel 4: Liposomal verkapseltes wasserdispergiertes Ferrofluid

5 g Phospholipon werden in einem Rundkolben in Chloroform gelöst und die organische Phase unter Vakuum am Rotationsverdampfter abgezogen, bis sich ein dünner lösungsmittelfreier Lipidfilm gebildet hat. 100 ml des wasserdispergierten Ferrofluidprodukts von Beispiel 1 wird unter leichtem Erwärmen zu dem Lipidfilm gegeben und für eine Stunde an einem mechanischen Schüttler bewegt, bis sich der Lipidfilm komplett von der Wand abgelöst hat und sich Liposomen gebildet haben. Das Präparat wird für 1-2 Minuten mit Ultraschall behandelt, um eine Grössenverteilung der Liposomen im Nanometerbereich zu erreichen. Das Präparat wird dann über eine Sephadex-G75 Säule von der nichtverkapselten Fraktion getrennt. Die Ferrofluid-Liposomendispersion kann mit isotonischer Kochsalzlösung auf die gewünschtge Konzentration eingestellt werden.

Beispiel 5: Wässerige Lösungen natürlicher oder synthetischer Proteine

1 ml einer wässerigen Lösung von Urodilatin (nephroprotektives Protein) wurde in 9 ml des ölbasierten Ferrofluids aus Beispiel 2 durch Zusatz von 1g Solutol® HS 15 und Rühren mittels Magnetrührer bei Raumtemperatur emulgiert. Man erhielt eine Wasser-in-Ferrofluid-Emulsion, welche in 30 ml einer 1%igen wässerigen Solutollösung mittels Magnetrührung emulgiert wurde. Es bildete sich eine proteinhaltige Wasser-in- Ferrofluid-in-Wasser-Emulsion mit Tröpfchengrößen im Bereich zwischen 3-50 Mikrometer.

Das Ferrofluid wird injiziert und transportiert das magnetunterstützte Urodilatin gezielt zum Wirkort Niere. Danach erfolgt Desorption und Separation der unbeladenen Partikel aus dem Blut über externe Blutwäsche, d.h. Ferropartikel werden durch magnetische Blutadsorber eingefangen und entsorgt.

Beispiel 6: Magnetischer Transport von DNA in einer Wasser-in-Öl-in-Wasser-Emulsion

1 ml einer isotonischen Salzlösung des Oligonucleotids single strand 24mer phosphorothioat (synthetisches DNA-Derivat) wurde in 9 ml des ölbasierten Ferrofluids aus Beispiel 2 durch Zusatz von 1g Solutol® HS 15 und starkes Rühren mittels Magnetrührer bei Raumtemperatur emulgiert. Man erhielt eine nicht langzeitstabile Wasser in Ferrofluid-Emulsion, welche in 30 ml einer 1 %igen wässerigen Solutollösung mittels Magnetrührung emulgiert wurde. Es bildete sich eine oligonucleotidhaltige Wasser in Ferrofluid-in-Wasser-Emulsion mit Tröpfchengrößen im Mikrometerbereich.

Beispiel 7: Magnetischer Transport von Zellen und Zellbestandteilen in einer Wasser-in-Öl-in-Wasser-Emulsion

1 ml einer isotonischen Salzlösung von gentechnisch veränderten Epithelzellen wurden in 9 ml in Beispiel 2 erhaltenen Ferrofluids durch Zusatz von 1g Solutol® HS 15 und Rühren mittels Magnetrührer bei Raumtemperatur emulgiert. Man erhielt eine nicht lang-zeitstabile Wasser in Ferrofluid-Emulsion, welche in 30 ml einer 1%igen wässerigen Solutollösung mittels Magnetrührung emulgiert wurde. Es bildete sich eine zellhaltige Wasser-in-Ferrofluid-in-Wasser-Emulsion mit Tröpfchengrößen im Mikrometerbereich.

Die erhaltene zellhaltige Ferrofluid-Emulsion ist beispielsweise zum zielgerichteten Transport und zur Plazierung der Zellen an einem speziellen Wirkort geeignet, wie z. B. zur Anheftung der Zellen an bestimmte Gefäßwandstellen (z. B. in Coronararterien nach PTCA).

**Patentansprüche**

1. Stabilisator-freies System für den Transport von Aktivstoffen in einem biologischen System bestehend aus einem oder mehreren Aktivstoff(en) sowie magnetischen Partikeln ausgewählt aus superparamagnetischen Metalloxiden und/oder Metallen, das **dadurch gekennzeichnet ist, dass** die Partikel auf zumindest einem Teil ihrer Oberfläche mit Aktivstoff(en) versehen sind.

2. System nach Anspruch 1, **dadurch gekennzeichnet, dass** das biologische System der menschliche oder tierische Körper, ein extrakorporales System, wie aus dem Körper eluierte Zellen/Zellkulturen und/oder außerhalb des Körpers befindliche Geräte, in denen Körperflüssigkeiten gereinigt, werden, ist.

3. System nach Anspruch 1 oder 2, **dadurch gekennzeichnet, dass** die magnetischen Oxide ausgewählt sind aus

$\gamma$-$Fe_2O_3$, $Fe_3O_4$, $MnFe_2O_4$, $NiFe_2O_4$, $CoFe_2O_4$ und beliebigen Gemischen daraus.

4. System nach einem der Ansprüche 1 bis 3, **dadurch gekennzeichnet, dass** magnetischen Partikel eine Teilchengröße von 1 bis 300 nm, vorzugsweise bis 100 nm, aufweisen.

5. System nach einem der Ansprüche 1 bis 4, **dadurch gekennzeichnet, dass** die Aktivstoffe ausgewählt sind aus Stoffen, die in den Körper eingebracht werden, als auch aus Stoffen, die aus ihm entfernt werden sollen.

6. System nach einem der Ansprüche 1 bis 5, **dadurch gekennzeichnet, dass** die Aktivstoffe wasserlöslich und/oder lipidlöslich sind und ausgewählt sind aus pharmazeutischen Wirkstoffen, synthetischen pharmazeutischen Wirkstoffen, natürlichen pharmazeutischen Wirkstoffen und Extrakten, natürlichen und rekombinanten Peptiden, Proteinen, Enzymen, Antikörpern und Antikörperfragmenten, endogenen biologischen Einheiten, wie lebenden und abgestorbene Zellen, Zellbestandteilen und Organellen, synthetischer und natürlicher DNA, Genen, Chromosomen, gentechnisch veränderten autologen oder heterologen Zellen, und xenobiotischen Einheiten, wie Bakterien, Viren, Mycoplasma, Pilze, und Sporen, wärmeleitfähigen Substanzen, wie Metalle, radiologisch wirksamen Stoffen, wie $\gamma$-Strahler, Aktivstoffe enthaltenden partikulären exogenen Einheiten, wie Liposomen, Mikrokapseln und Nanopartikeln, sowie beliebige Gemische der voranstehenden.

7. System nach einem der Ansprüche 1 bis 6, **dadurch gekennzeichnet, dass** die wasserlöslichen Aktivstoffe ausgewählt sind aus geminalen Bisphosphonsäuren und/oder deren physiologisch verträglichen Salzen der allgemeinen Formel I

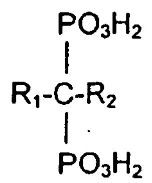

Formel I

in der ist

$R_1$ ein linearer oder verzweigter Alkylrest mit 1 bis 10 Kohlenstoffatomen, der gegebenenfalls substituiert sein kann durch Substituenten wie Aminogruppen, N-Mono- bzw. N-Dialkylaminogruppen, wobei die Alkylgruppen 1 bis 5 C-Atome und/oder SH-Gruppen enthalten können, oder ein substituierter oder unsubstituierter carbo- oder heterocyclischer Arylrest, der ggf. ein oder mehrere Heteroatome und als Substituenten verzweigte und unverzweigte Alkylreste mit 1 bis 6 C-Atomen, freie oder mono- resp. dialkylierte Aminogruppen mit 1 bis 6 C-Atomen oder Halogenatome aufweisen kann, ist und
$R_2$ gleich OH, ein Halogenatom, vorzugsweise Cl, H oder $NH_2$.

8. System nach Anspruch 7, **dadurch gekennzeichnet, dass** das Bisphosponat ausgewählt ist aus 3-(Methyt-pentylamino)-1-hydroxypropan-1,1-diphosphonsäure (Ibandronsäure), 1-Hydroxyethan-1,1-diphosphonsäure (Etidronsäure), Dichlormethandiphosphonsäure (Clodronsäure), 3-Amino-1-hydroxypropan-1,1-diphosphonsäure (Pamidronsäure), 4-Amino-1-hydroxybutan-1,1-diphosphonsäure (Alendronsäure), 2-(3-Pyridin)-1-hydroxyethan-1,1-diphosphonsäure (Risedronsäure), 4-Chlorphenylthiomethan-1,1-diphosphonsäure (Tiludronsäure), Pyrimidinyl-1-hydroxyethan-1,1-diphosphonsäure (Zoledronsäure), Cycloheptylaminomethan-1,1-diphosphonsäure (Cimadronsäure), 6-Amino-1-hydroxyhexan-1,1-diphosphonsäure (Neridronsäure), 3-(N,N-Dimethylamino)-1-hydroxypropan-1,1-diphosphonsäure (Olpadronsäure), 3-Pyrrol-1-hydroxypropan-1,1-diphosphonsäure und/oder 2-Pyrimidazol-1-hydroxyethan-1,1-diphosphonsäure (Minodronsäure) sowie deren physiologisch verträglichen Salzen.

9. System nach einem der Ansprüche 1 bis 8, **dadurch gekennzeichnet, dass** es als Suspension, Emulsion oder liposomales System vorliegt und in eine pharmazeutische Zubereitung für die orale, parenterale, intravenöse, inhalative und/oder topische Applikation überführt wird.

10. Verfahren zur Herstellung eines Systems für den Transport von Aktivstoffen nach einem der Ansprüche 1 bis 9, **dadurch gekennzeichnet, dass** ein wasserlöslicher oder in Wasser suspendierbarer Aktivstoff und eine in Wasser lösliche Vorstufe der magnetischen Partikel in Wasser gelöst und die magnetischen Partikel durch Ausfällung gebildet, wobei die magnetischen Partikel beladen mit dem Aktivstoff als Feststoff ausfallen.

**11.** Verfahren zur Herstellung eines Systems für den Transport von Aktivstoffen nach einem der Ansprüche 1 bis 9,- **dadurch gekennzeichnet, dass** die magnetischen Partikel in eine Lösung oder Suspension des Aktivstoffs in Wasser oder einer anderen Flüssigkeit gegeben werden.

**12.** Verwendung des System für den Transport von Aktivstoffen in einem biologischen System nach einem der Ansprüche 1 bis 9 zur Herstellung eines Arzneimittels für den zielgerichtete Transport pharmazeutischen Wirkstoffen in dem biologischen System.

**13.** Verwendung des System für den Transport von Aktivstoffen in einem biologischen System nach einem der Ansprüche 1 bis 9 zur Herstellung eines Arzneimittels für die Anreicherung von Aktivstoffen in dem biologischen System an vorgegebenen Orten.

## Claims

**1.** Stabilizer-free system for transporting active substances in a biological system consisting of one or more active substance(s) and magnetic particles selected from superparamagnetic metal oxides and/or metals, which is **characterized in that** the particles are provided with active substance(s) on at least part of their surface.

**2.** System according to Claim 1, **characterized in that** the biological system is the human or animal body, an extracorporeal system such as cells/cell cultures eluted from the body and/or devices outside the body in which body fluids are purified.

**3.** System according to Claim 1 or 2, **characterized in that** the magnetic oxides are selected from $\gamma$-$Fe_2O_3$, $Fe_3O_4$, $MnFe_2O_4$, $NiFe_2O_4$, $CoFe_2O_4$ and any mixtures thereof.

**4.** System according to one of Claims 1 to 3, **characterized in that** the magnetic particles have a particle size of 1 to 300 nm, preferably up to 100 nm.

**5.** System according to one of Claims 1 to 4, **characterized in that** the active substances are selected from substances which are introduced into the body and from substances which are to be removed from it.

**6.** System according to one of Claims 1 to 5, **characterized in that** the active substances are water-soluble and/or lipid-soluble and are selected from pharmaceutical active ingredients, synthetic pharmaceutical active ingredients, natural pharmaceutical active ingredients and extracts, natural and recombinant peptides, proteins, enzymes, antibodies and antibody fragments, endogenous biological units such as living and dead cells, cell components and organelles, synthetic and natural DNA, genes, chromosomes, genetically modified autologous or heterologous cells, and xenobiotic units such as bacteria, viruses, mycoplasma, fungi and spores, heat-conductive substances such as metals, radiologically active substances such as $\gamma$-radiators, particulate exogenous units containing active substances, such as liposomes, microcapsules and nanoparticles, and any mixtures of the above.

**7.** System according to one of Claims 1 to 6, **characterized in that** the water-soluble active substances are selected from geminal bisphosphonic acids and/or the physiologically compatible salts thereof of the general formula I

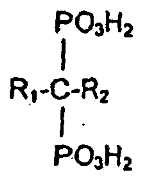

Formula 1

in which

$R_1$ is a linear or branched alkyl radical having 1 to 10 carbon atoms, which is unsubstituted or substituted with substituents such as amino groups, N-mono- or N-dialkylamino groups, wherein the alkyl groups may contain

1 to 5 carbon atoms and/or SH groups, or a substituted or unsubstituted carbocyclic or heterocyclic aryl radical which may have, where appropriate, one or more heteroatoms and, as substituents, branched and unbranched alkyl radicals having 1 to 6 carbon atoms, free or mono-or respectively dialkylated amino groups having 1 to 6 carbon atoms or halogen atoms, and

$R_2$ is OH, a halogen atom, preferably Cl, H or $NH_2$.

**8.** System according to Claim 7, **characterized in that** the bisphosphonate is selected from 3-(methylpentyl-amino)-1-hydroxypropane-1,1-diphosphonic acid (ibandronic acid), 1-hydroxyethane-1,1-diphosphonic acid (etidronic acid), dichloromethanediphosphonic acid (clodronic acid), 3-amino-1-hydroxypropane-1,1-diphosphanic acid (pamidronic acid), 4-amino-1-hydroxybutane-1,1-diphosphonic acid (alendronic acid), 2-(3-pyridine)-1-hydroxyethane-1,1-diphosphonic acid (risedronic acid), 4-chlorophenylthiomethane-1,1-diphosphonic acid (tiludronic acid), pyrimidinyl-1-hydroxyethane-1,1-diphosphonic acid (zoledronic acid), cycloheptylaminomethane-1,1-diphosphonic acid (cimadronic acid), 6-amino-1-hydroxyhexane-1,1-diphosphonic acid (neridronic acid), 3-(N,N-dimethylamino)-1-hydroxypropane-1,1-diphosphonic acid (olpadronic acid), 3-pyrrole-1-hydroxypropane-1,1-diphosphonic acid and/or 2-pyrimidazole-1-hydroxyethane-1,1-diphosphonic acid (minodronic acid) and the physiologically compatible salts thereof.

**9.** System according to one of Claims 1 to 8, **characterized in that** it exists as a suspension, emulsion or liposomal system and is transferred to a pharmaceutical preparation for oral, parenteral, intravenous, inhalative and/or topical administration.

**10.** Method for preparing a system for transporting active substances according to one of Claims 1 to 9, **characterized in that** a water-soluble or water-suspendable active substance and a water-soluble precursor of the magnetic particles are dissolved in water and the magnetic particles are formed by precipitation, wherein the magnetic particles precipitate as solids loaded with the active substance.

**11.** Method for preparing a system for transporting active substances according to one of Claims 1 to 9, **characterized in that** the magnetic particles are introduced into a solution or suspension of the active substance in water or another liquid.

**12.** Use of the system to prepare a medicament for transporting active substances in a biological system according to one of Claims 1 to 9 for the targeted transport of pharmaceutical active ingredients in the biological system.

**13.** Use of the system to prepare a medicament for transporting active substances in a biological system according to one of Claims 1 to 9 for concentrating active substances in the biological system at predetermined locations.

**Revendications**

**1.** Système dépourvu de stabilisateur destiné au transport de matières actives dans un système biologique, constitué d'une ou de plusieurs matières actives ainsi que de particules magnétiques choisies parmi les oxydes métalliques super-paramagnétiques et/ou les métaux, **caractérisé en ce que** les particules sont dotées de matières actives sur au moins une partie de leur surface.

**2.** Système selon la revendication 1, **caractérisé en ce que** le système biologique est le corps humain ou animal, un système extracorporel, comme des cellules/cultures cellulaires éluées d'un corps, et/ou des appareils se trouvant en dehors du corps, dans lesquels des liquides physiologiques sont purifiés.

**3.** Système selon la revendication 1 ou 2, **caractérisé en ce que** les oxydes magnétiques sont choisis parmi $\gamma$-$Fe_2O_3$, $Fe_3O_4$, $MnFe_2O_4$, $NiFe_2O_4$, $CoFe_2O_4$ et des mélanges quelconques de ceux-ci.

**4.** Système selon l'une des revendications 1 à 3, **caractérisé en ce que** les particules magnétiques présentent une granulométrie de 1 à 300 nm, de préférence jusqu'à 100 nm.

**5.** Système selon l'une des revendications 1 à 4, **caractérisé en ce que** les matières actives sont choisies aussi bien parmi les matières qui sont introduites dans le corps que parmi les matières qui doivent en être retirées.

**6.** Système selon l'une des revendications 1 à 5, **caractérisé en ce que** les matières actives sont hydrosolubles et/ou

liposolubles et sont choisies parmi les principes actifs pharmaceutiques, les principes actifs pharmaceutiques synthétiques, les principes actifs pharmaceutiques et extraits naturels, les peptides naturels et recombinants, les protéines, les enzymes, les anticorps et fragments d'anticorps, les unités biologiques endogènes, comme les cellules vivantes et les cellules mortes, les composants cellulaires et les organelles, l'ADN synthétique et naturel, les gènes, les chromosomes, les cellules autologues ou hétérologues génétiquement modifiées, et les entités xénobiotiques, comme les bactéries, les virus, les mycoplasmes, les champignons, les spores, les substances thermoconductrices comme les métaux, les matières radiologiquement actives comme les émetteurs de rayons $\gamma$, les entités exogènes particulaires contenant des matières actives comme les liposomes, les microcapsules et nanoparticules, ainsi que des mélanges quelconques des matières actives précédentes.

7. Système selon l'une des revendications 1 à 6, **caractérisé en ce que** les matières actives hydrosolubles sont choisies parmi les acides bisphosphoniques géminaux et/ou leurs sels physiologiquement acceptables de formule générale I

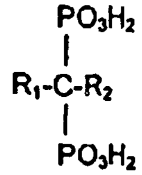

Formule I

dans laquelle

$R^1$ est un radical alkyle linéaire ou ramifié ayant de 1 à 10 atomes de carbone, qui peut être le cas échéant substitué par des substituants tels que des groupes amino, des groupes N-mono- ou N-dialkylamino, où les groupes alkyles peuvent contenir de 1 à 5 atomes de C et/ou des groupes SH, ou un radical aryle carbo- ou hétérocyclique substitué ou non substitué, qui peut présenter le cas échéant un ou plusieurs hétéroatomes et, comme substituants, des radicaux alkyles ramifiés et non ramifiés ayant de 1 à 6 atomes de C, des groupes amino libres ou mono- ou dialkylés ayant de 1 à 6 atomes de C ou des atomes d'halogène, et
$R_2$ est égal à OH, un atome d'halogène, de préférence Cl, H ou $NH_2$.

8. Système selon la revendication 7, **caractérisé en ce que** le bisphosphonate est choisi parmi l'acide 3-(méthylpentylamino)-1-hydroxypropane-1,1-diphosphonique (acide ibandronique), l'acide 1-hydroxyéthane-1, 1-diphosphonique (acide étidronique), l'acide dichlorométhanediphosphonique (acide clodronique), l'acide 3-amino-1-hydroxypropane-1,1-diphosphonique (acide pamidronique), l'acide 4-amino-1-hydroxybutane-1,1-diphosphonique (acide alendronique), l'acide 2-(3-pyridine)-1-hydroxyéthane-1,1-diphosphonique (acide risédronique), l'acide 4-chlorophénylthiométhane-1,1-diphosphonique (acide tiludronique), l'acide pyrimidinyl-1-hydroxyéthane-1,1-disphosphonique (acide zolédronique), l'acide cycloheptylaminométhane-1,1-diphosphonique (acide cimadronique), l'acide 6-amino-1-hydroxyhexane-1,1-diphosphonique (acide néridronique), l'acide 3-(N,N-diméthylamino)-1-hydroxypropane-1,1-diphosphonique (acide olpadronique), l'acide 3-pyrrol-1-hydroxypropane-1,1-diphosphonique et/ou l'acide 2-pyrimidazol-1-hydroxyéthane-1,1-diphosphonique (acide minodronique) ainsi que leurs sels physiologiquement acceptables.

9. Système selon l'une des revendications 1 à 8, **caractérisé en ce qu'**il se présente sous forme de suspension, d'émulsion ou de système liposomal et est transformé en une composition pharmaceutique pour l'administration orale, parentérale, intraveineuse, inhalative et/ou topique.

10. Procédé de fabrication d'un système destiné au transport de matières actives selon l'une des revendications 1 à 9, **caractérisé en ce qu'**une matière active hydrosoluble ou pouvant être mise en suspension dans l'eau et un précurseur soluble dans l'eau des particules magnétiques sont dissoutes dans l'eau et les particules magnétiques sont formées par précipitation, les particules magnétiques chargées de matière active sous forme solide se précipitant.

11. Procédé de fabrication d'un système destiné au transport de matières actives selon l'une des revendications 1 à 9,

**caractérisé en ce que** les particules magnétiques sont mélangées avec une solution ou suspension de matière active dans de l'eau ou dans un autre liquide.

12. Utilisation du système destiné au transport de matières actives dans un système biologique selon l'une des revendications 1 à 9, pour la fabrication d'un médicament pour le transport ciblé de principes actifs pharmaceutiques dans le système biologique.

13. Utilisation du système destiné au transport de matières actives dans un système biologique selon l'une des revendications 1 à 9, pour la fabrication d'un médicament pour l'enrichissement en matières actives dans un système biologique sur des sites prédéterminés.